# EUROPÄISCHE PATENTANMELDUNG

(11) **EP 2 031 532 A2**
(43) Veröffentlichungstag der Anmeldung: **04.03.2009**
(21) Anmeldenummer: 08159444.2
(22) Anmeldetag: 01.07.2008
(51) Int. Cl.: G06F 19/00

(54) **Anordnung und Verfahren für die Fernprogrammierung eines persönlichen medizinischen Gerätes**

(30) Priorität: 28.07.2007 DE 102007035534
(71) Anmelder: BIOTRONIK CRM Patent AG, 6341 Baar (CH)
(72) Erfinder: Reinke, Joachim, 10439 Berlin (DE)
(74) Vertreter: Lindner-Vogt, Karin L.

(57) **Zusammenfassung**

Die Erfindung besteht in einer Anordnung zur sicheren Fernprogrammierung eines Implantats. Hierfür ist ein TAN-Server vorgesehen, bei dem sich ein Benutzer zunächst akkreditiert und der dann auf eine Anfrage hin eine TAN generiert und diese zum einen dem Benutzer zustellt und zum anderen einem dem umzuprogrammierenden Implantat zugeordneten Patientengerät.

## Beschreibung

Die Erfindung betrifft eine Anordnung und ein Verfahren für die Fernprogrammierung eines persönlichen medizinischen Gerätes, insbesondere eines implantierbaren medizinischen Gerätes, wie beispielsweise einem Herzschrittmacher, einem Defibrillator oder dergleichen.

Im Zusammenhang mit Herzschrittmachern oder Defibrillatoren im Verbund mit einem Servicecenter ist es möglich und auch hinlänglich bekannt, seitens eines Herzschrittmachers oder Defibrillators gewonnene medizinische, physiologische oder Betriebsdaten zu dem zentralen Servicecenter zu übertragen, um diese Daten dort auszuwerten und über eine entsprechende Benutzerschnittstelle einem betreuenden Arzt zur Verfügung zu stellen.

Einige Funktionen solcher Implantate sind durch Software oder Firmenware gesteuert und daher programmierbar. Dabei kommt es immer wieder vor, dass nach anfänglicher Programmierung kurz vor, während oder nach der Implantation weitere Programmierungen oder Umprogrammierungen wünschenswert sind, um das Implantat besser auf möglicherweise inzwischen veränderte Gesundheitszustände eines Patienten einstellen zu können oder die Leistungsfähigkeit des Implantats anderweitig zu erhöhen. Häufig geschieht ein derartiges Programmieren oder Umprogrammieren dadurch, dass ein Arzt zu einem jeweiligen Implantat mit Hilfe eines Programmiergerätes eine kurzreichweitige, drahtlose Datenverbindung herstellt und das Implantat im Angesicht des Patienten programmiert.

Eine Programmierung oder Umprogrammierung des Implantats kann jedoch grundsätzlich auch aus der Ferne, beispielsweise über das zentrale Servicecenter, geschehen. Hierzu kann eine Datenverbindung zwischen dem Servicecenter und einem Patientengerät hergestellt werden, welches sich üblicherweise in der Nähe eines Patienten befindet und eine bidirektionale Datenverbindung zwischen dem Implantat und dem Patientengerät herstellen kann. Die Verbindung zwischen Servicecenter und Patientengerät kann dabei drahtlos separat gebunden, beispielsweise über das Telefonnetz, das Internet oder ähnliche Datenleitungen, ausgebildet sein.

Hierbei besteht grundsätzlich das Problem sicherzustellen, dass das jeweilige Im-plantat oder auch das Patientengerät als persönliches medizinisches Gerät nicht infolge einer fehlerhaften Datenübertragung oder gar missbräuchlich umprogrammiert werden kann.

Zur Lösung dieses Problems ist es beispielsweise gemäß US 6,442,432 vorgesehen, Programmieraufträge für das Programmieren oder Umprogrammieren eines Implantats mit einem Public-Key-Verschlüsselungsverfahren, wie beispielsweise PGP, zu verschlüsseln beziehungsweise zu verifizieren.

Der vorliegenden Erfindung liegt die Aufgabe zugrunde, auf alternative Weise die Verifikation sicherzustellen, dass ein Programmierauftrag an ein persönliches Gerät, wie ein Implantat oder ein Patientengerät, in jedem Fall authentisch ist und von befugter Quelle stammt.

Erfindungsgemäß wird diese Aufgabe durch eine Anordnung der eingangs genannten Art gelöst, welche als Komponente neben dem persönlichen Gerät wenigstens zusätzlich einen TAN-Server aufweist. Dieser TAN-Server besitzt eine Datenbank, die einander zugeordnete Einträge zu jeweils einer Benutzererkennung, jeweils wenigstens einer Gerätekennung und jeweils wenigstens einer Nachrichtenadresse mitteilt. Die Benutzerkennung identifiziert einen Benutzer, beispielsweise einen Arzt. Die Gerätekennung identifiziert ein dem Benutzer zugeordnetes, beispielsweise von einem Arzt betreutes, persönliches Gerät wie beispielsweise ein Implantat oder Patientengerät. Die Nachrichtenadresse ist die Adresse, beispielsweise die Telefonnummer oder e-mail-Adresse, eines adressierbaren persönlichen Kommunikationsgerätes wie beispielsweise eines Handys, eines Faxgerätes oder eines e-mail-Clients. Der TAN-Server weist außerdem eine TAN-Benutzerschnittstelle auf, die ausgebildet ist, Eingaben eines Benutzers entgegenzunehmen und die Eingaben einer Benutzerkennung zuzuordnen und derart zugeordnet an den TAN-Server weiterzuleiten. Außerdem besitzt der TAN-Server eine Nachrichtenschnittstelle, über die der TAN-Server eine Nachricht an ein adressierbares Kommunikationsgerät (Fax, e-mail-Clients, Handy (SMS)) eines Benutzers, das durch seine Nachrichten adressidentifiziert ist, senden kann. Weiterer Bestandteil des TAN-Servers ist eine Datenkommunikationsschnittstelle für eine wenigstens mittelbare Verbindung zur Übertragung von Daten an das programmierbare persönliche Gerät.

Der TAN-Server ist ausgebildet, eine einer Benutzerkennung zugeordnete TAN-Anfrage zu empfangen und anschließend eine TAN-Nachricht zu generieren. Die TAN-Nachricht enthält eine eindeutige Transaktionsnummer (TAN) zur Authentifizierung einer Transaktion, wie z. B. der Umprogrammierung des persönlichen Gerätes. Diese TAN-Nachricht sendet der TAN-Server über die Nachrichtenschnittstelle an das durch die über die Datenbankeinträge der Benutzerkennung des anfragenden Benutzers zugeordnete Kommunikationsgerät und über die Datenkommunikationsschnittstelle an ein durch die Datenbankeinträge der Benutzerkennung zugeordnetes und gegebenenfalls vom Benutzer ausgewähltes, durch seine Gerätekennung identifiziertes persönliches Gerät.

Das programmierbare persönliche Gerät besitzt wenigstens eine Datenkommunikationsschnittstelle zum wenigstens mittelbaren Verbinden des persönlich programmierbaren Gerätes mit dem TAN-Server und einen TAN-Speicher um eine seitens des TAN-Servers empfangene TAN zu speichern. Das programmierbare persönliche Gerät ist ausgebildet, eine seitens des TAN-Servers empfangene TAN in dem TAN-Speicher zu speichern.

Durch eine derartige Anordnung ist sichergestellt, dass einer einem persönlichen medizinischen Gerät also insbesondere einem Patientengerät oder einem Implantat zugeordnete Person eine TAN zugestellt wird, die gleichzeitig auch in dem persönlichen Gerät gespeichert wird. Übertragen wird dabei nur die TAN selbst, sodass ein Empfänger, der beispielsweise die an dem Benutzer versandte TAN abfängt, nicht weiß, zu welchem persönlichen Gerät diese gehört.

Die Sicherheit der Anordnung lässt sich steigern, wenn der TAN-Server weiterhin ausgebildet ist, zum Zwecke der Akkreditierung eines Benutzers zunächst über die TAN-Benutzerschnittstelle eine Benutzerkennung und eine Nachrichtenadresse eines Kommunikationsgerätes zu empfangen und daraufhin eine Nachricht eines Kommunikationsgerätes zu versenden. Hierbei ist der TAN-Server weiterhin dazu ausgebildet, über die TAN-Benutzerschnittstelle eine Nachricht zu empfangen und diese Nachricht mit der an das Kommunikationsgerät gesandten Nachricht zu vergleichen und für den Fall der Übereinstimmung die Benutzerkennung zusammen mit der Nachrichtenadresse des Kommunikationsgerätes als einander zugeordnete Einträge in der Datenbank zu speichern. Ein Benutzer, der sich beim TAN-Server akkreditieren möchte, muss also seine Benutzerkennung zusammen mit einer Adresse eines Kommunikationsgerätes an den TAN-Server senden. Zur Verifikation von Benutzer und Adresse des Kommunikationsgerätes (Nachrichtenadresse) schickt der TAN-Server daraufhin eine Nachricht an das Kommunikationsgerät. Der Benutzer muss diese Nachricht wieder an den TAN-Server senden, um die Akkreditierung abzuschließen. Wenn die von dem Benutzer zusammen mit einer Benutzerkennung an den TAN-Server gesandte Nachricht mit der zwischengespeicherten, zuvor an das dem Benutzer über seine Benutzerkennung zugeordnete Kommunikationsgerät gesandten Nachricht übereinstimmt, ist der Benutzer über seine Benutzerkennung und die zugehörige Nachrichtenadresse eines Kommunikationsgerätes bei dem TAN-Server akkreditiert und kann später wie zuvor beschrieben, eine TAN-Anfrage durchführen.

Nach der TAN-Anfrage hält der Benutzer eine valide TAN in den Händen, die gleichzeitig in dem zu programmierenden persönlichen Gerät gespeichert ist.

Vorzugsweise ist die TAN-Benutzerschnittstelle des TAN-Servers auf einem von dem TAN-Server räumlich entfernten Computer lauffähig und über ein Datennetz mit dem TAN-Server verbunden. Auf diese Weise ist es möglich, einen zentralen TAN-Server vorzusehen

Vorzugsweise weist die Anordnung als weitere Komponente ein vom TAN-Server verschiedenes Servicecenter auf. Dieses besitzt zum einen eine Datenkommunikationsschnittstelle für eine Datenkommunikation mit dem persönlichen Gerät. Eine weitere, mit dem Servicecenter verbundene Service-Benutzerschnittstelle, ist so ausgebildet, dass sie es erlaubt, Programmieraufträge für das programmierbare persönliche Gerät zusammen zu stellen, ein Absenden eines zusammengestellten Programmierauftrags auszulösen und eine manuelle Anfrage für eine TAN zu einem Programmierauftrag abzufragen und eine entsprechende entgegengenommene TAN einem Programmierauftrag hinzuzufügen. Das Servicecenter ist weiterhin ausgebildet, einen freigegebenen beziehungsweise abgesandten Programmierauftrag zusammen mit einer über die Service-Benutzerschnittstelle freigegebenen TAN an das persönliche Gerät zu senden. Das persönliche Gerät - also das Programmiergerät oder das Patientengerät - ist dazu ausgebildet, einen zuvor seitens des TAN-Servers empfangene, gespeicherte TAN mit einer zusammen mit einem Programmierauftrag seitens des Servicecenters empfangenen TAN zu vergleichen und den Programmierauftrag nur auszuführen oder beispielsweise an ein Implantat weiterzuleiten, wenn beide TAN identisch sind.

Die Service-Benutzerschnittstelle kann als Fernprogrammieranwendung ausgebildet sein oder mit einer Fernprogrammieranwendung verbunden sein, die auf einem entfernt lauffähigen Gerät zur Ausführung kommen kann. Damit kann die Fernprogrammieranwendung beispielsweise auf dem Computer eines Arztes installiert sein.

Die Service-Benutzerschnittstelle ist vorzugsweise so ausgebildet, dass sie eine TAN-Anfrage eines Benutzers zusammen mit der Benutzerkennung dieses Benutzers entgegen nehmen kann. Das Servicecenter weist eine zweite Datenkommunikationsschnittstelle auf, die ausgebildet ist, einen Datenaustausch vom Servicecenter zum TAN-Server zu ermöglichen. Das Servicecenter ist ausgebildet, eine über die Service-Benutzerschnittstelle abgefangene TAN-Anfrage an den TAN-Server weiterzuleiten. Damit braucht der Benutzer die TAN-Anfrage nicht direkt an den TAN-Server zu richten, sondern kann die TAN-Anfrage zusammen mit einem Programmierauftrag über die Service-Benutzerschnittstelle generieren und absenden. In analoger Weise kann die TAN-Benutzerschnittstelle mit einer TAN-Serveranwendung verbunden sein, die ebenfalls entfernt vom TAN-Server lauffähig ist und es einem Benutzer ermöglicht, von seinem eigenen Computer aus eine TAN-Anfrage zusammen mit seiner Benutzerkennung einzugeben und dem TAN-Server zukommen zu lassen.

Die hier vorgestellten Abläufe für das Akkreditieren eines Benutzers auf dem TAN-Server, für die Anfrage zum Erhalt einer TAN und deren Generierung sowie schließlich die TANgeschützte Fernprogrammierung eines Implantats sind Gegenstände von Verfahren, die ebenfalls eine Lösung der eingangs genannten Aufgabe darstellen.

Die Erfindung soll nun anhand eines Ausführungsbeispiels mit Bezug auf die Figuren näher erläutert werden. Diese zeigen in
Fig. 1: Eine Anordnung zur Fernprogrammierung eines Implantats; und
Fig. 2: Blocksschaltbilder der Komponenten der Anordnung aus Figur 1.

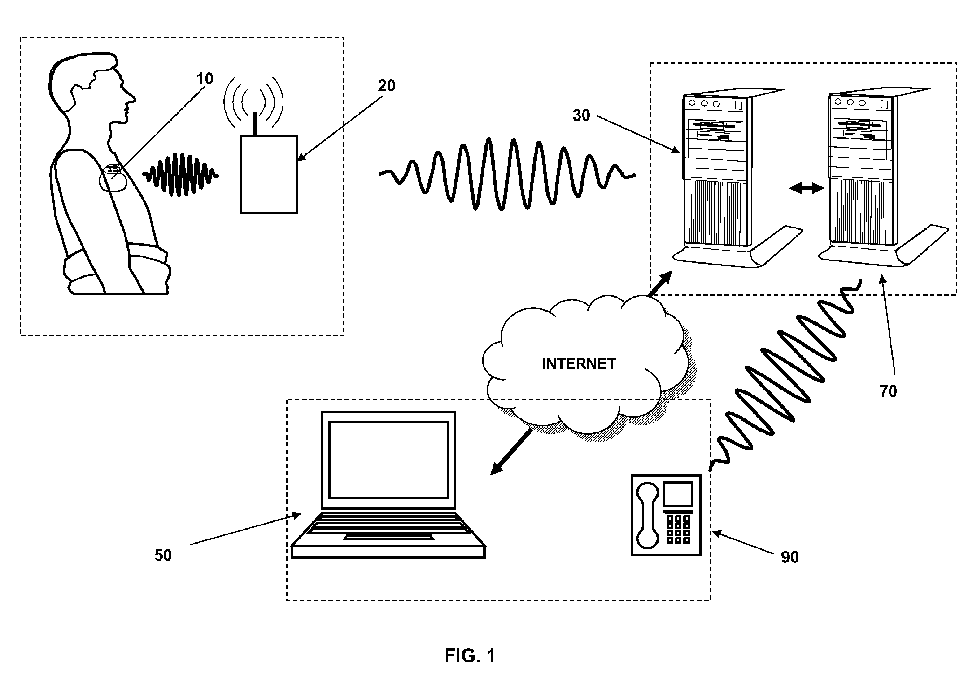

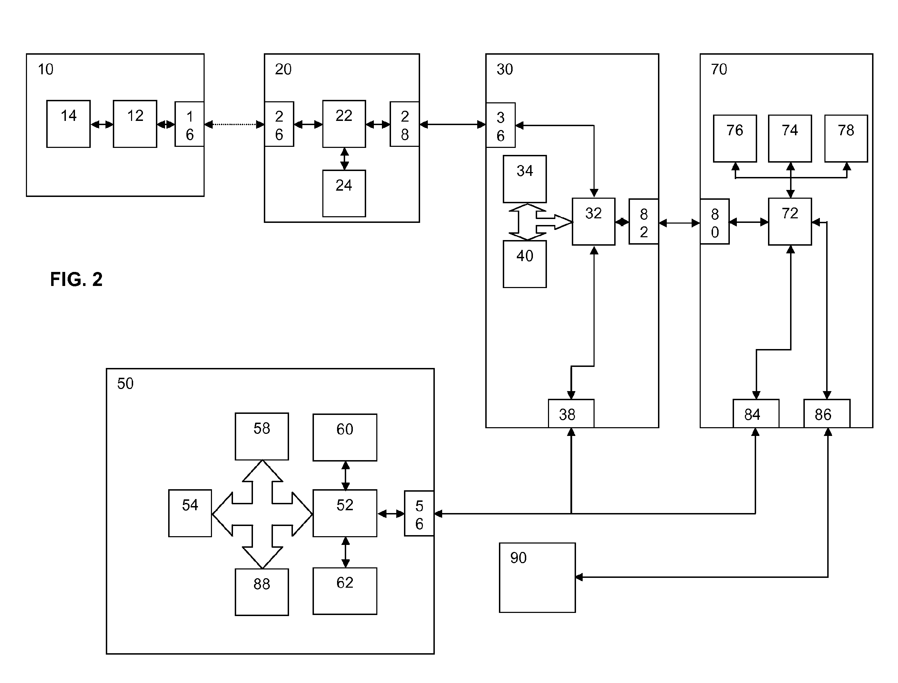

Figur 1 zeigt die Komponenten einer beispielhaften Anordnung zur Fernprogrammierung von Implantaten. Eine Komponente ist das Implantat 10 selbst. Dieses besitzt eine Steuerung 12 (siehe Figur 2), die sowohl mit einem Speicher 14 als auch mit einer Telemetrieeinheit 16 verbunden ist. Die Telemetrieeinheit 16 ermöglicht eine drahtlose, bidirektionale Datenübertragung von und zu dem Implantat 10.

Eine weitere Komponente ist ein Patientengerät 20, das ebenfalls eine Steuerung 22 aufweist, die mit einem Speicher 24 und einer ersten Datenkommunikationsschnittstelle 26 für eine bidirektionale, Datenübertragung von und zu dem Implantat 10 verbunden ist, sowie mit einer zweiten Datenkommunikationsschnittstelle 28, über die das Patientengerät 20 bidirektional Daten mit einem zentralen Servicecenter 30 austauschen kann.

Das zentrale Servicecenter 30 weist neben einer Steuerung 32 eine zentrale Datenbank 34 und eine erste Datenkommunikationsschnittstelle 36 auf, über die das Servicecenter 30 bidirektional Daten mit dem Patientengerät 20 austauschen kann. Außerdem weist das zentrale Servicecenter 30 eine Fernprogrammierservereinheit 40 auf, die sowohl mit der Steuereinheit 32 des Servicecenters 30 verbunden ist als auch mit einer Internetschnittstelle 38 zum Anschluss eines Fernprogrammierclients 50.

Der Fernprogrammierclient 50 besitzt ebenfalls eine zentrale Steuereinheit 52, die mit einem Speicher 54 und einer Datenkommunikationsschnittstelle 56 für eine bidirektionale Datenkommunikation mit dem Servicecenter 30 verbunden ist. Außerdem weist der Fernprogrammierclient 50 eine Fernprogrammieranwendung 58 auf, die mit Hilfe der zentralen Steuereinheit 52 und des Speichers 54 ausgeführt werden kann und entweder als standalone Lösung ausschließlich auf dem Fernprogrammierclient 50 lauffähig ist oder als Serveranwendung teilweise oder ganz auch auf das zentrale Servicecenter 30 und deren Fernprogrammierservereinheit 40 zurückgreift. Die Fernprogrammieranwendung 58, Anzeige 60 und Eingabeeinheit 62 bilden eine Service-Benutzerschnittstelle, die über die Internetschnittstelle 38 mit dem Servicecenter 30 verbunden ist.

Die Fernprogrammieranwendung 58 erlaubt es, auf dem Fernprogrammierclient 50 Programme zusammenzustellen, die für das Implantat 10 und dessen Steuerung 12 ausführbar sind. Die so zusammengestellten Programme können in Form eines Programmierauftrages von dem zentralen Servicecenter 30 über das Patientengerät 20 zum Implantat 10 übertragen werden.

Um von der Fernprogrammieranwendung 58 generierte Darstellungen anzuzeigen, weist der Fernprogrammierclient 50 eine Anzeige 60 auf. Damit ein Benutzer, beispielsweise ein Arzt, Eingaben für die Fernprogrammierung des Implantats 10 machen kann, ist außerdem eine Eingabeeinheit 62 vorgesehen.

Räumlich befinden sich das Implantat 10 (ohnehin) und das Patientengerät 20 in der Nähe eines Patienten. Das zentrale Servicecenter 30 ist an einem zentralen Ort angeordnet. Der Fernprogrammierclient 50 befindet sich in der Nähe eines Arztes und kann vom Servicecenter 30 weit entfernt sein.

Weiterer Bestandteil der Anordnung zur TAN-geschützten Fernprogrammierung eines medizinischen Implantats sind ein vom Servicecenter 30 unabhängiger TAN-Server 70, sowie ein persönliches Kommunikationsgerät 90 beispielsweise ein e-mail-Client, ein Mobiltelefon oder ein Faxgerät eines Arztes, welches seitens des TAN-Servers 70 ausgesandte Nachrichten empfangen kann.
Der TAN-Server 70 besitzt eine TAN-Server-Steuereinheit 72, die sowohl mit einer Datenbank 74 als auch mit einem TAN-Generator 76 und einer Vergleichseinheit 78 verbunden ist.

Eine Datenkommunikationsschnittstelle 80 erlaubt den Datenaustausch mit dem Servicecenter 30. Dieses weist eine entsprechende Datenschnittstelle 82 auf. Eine weitere Datenkommunikationsschnittstelle 84 erlaubt den bidirektionalen Datenaustausch mit einem persönlichen Computer eines Arztes, insbesondere mit dem Fernprogrammierclient 50. Schließlich ist eine dritte Datenkommunikationsschnittstelle 86 vorgesehen, über die der TAN-Server 70 mit dem Kommunikationsgerät 90 verbunden ist. Die dritte Datenkommunikationsschnittstelle 86 bildet die Nachrichtenschnittstelle im Sinne der Erfindung.

Auf dem Fernprogrammierclient 50 ist außerdem eine TAN-Serveranwendung 88 installiert, die einen Zugriff auf den TAN-Server 70 von Fernprogrammierclient 50 aus erlaubt. Die TAN-Serveranwendung 88, die Anzeige 60 und Eingabeeinheit 62 bilden eine TAN-Benutzerschnittstelle, die über die Datenkommunikationsschnittstelle 84 mit dem TAN-Server 70 verbunden ist.

Der Speicher 24 des Patientengerätes 20 ist ausgebildet, eine vom TAN-Server 70 direkt oder - wie im dargestellten Fall - mittels des Servicecenters 30 empfangene TAN zu speichern. Die Steuerung 22 des Patientengerätes 20 ist darüber hinaus ausgebildet, eine im Speicher 24 gespeicherte TAN mit einer TAN in einem empfangen Programmierauftrag zu vergleichen und einen empfangenen Programmierauftrag nur dann an das Implantat 10 weiterzuleiten, wenn beide TAN übereinstimmen.

Für das sichere Generieren einer derartigen TAN ist der TAN-Server 70 vorgesehen.

Ein Benutzer, der das Implantat 10 fernprogrammieren möchte, muss sich bei dem TAN-Server 70 zunächst akkreditieren. Dazu nutzt er die TAN-Serveranwendung 88 auf dem Fernprogrammierclient 50 und gibt seine Benutzerkennung zusammen mit einer Nachrichtenadresse für sein persönliches Kommunikationsgerät 90 ein. Der TAN-Server 70 generiert daraufhin eine Testnachricht und sendet diese an die ihm zuvor übersandte Nachrichtenadresse. Ein Arzt kann nun diese Nachrichtenadresse über die TAN-Serveranwendung 88 zusammen mit seiner Benutzerkennung eingeben und eine entsprechende Nachricht an den TAN-Server 70 senden. Im TAN-Server 70 vergleicht die Vergleichseinheit 78 die zuvor abgesandte Nachricht mit der zuletzt empfangenen Nachricht und veranlasst, dass im Falle der Übereinstimmung die Benutzerkennung zusammen mit der Nachrichtenadresse sowie einer Gerätekennung, die ein Patientengerät 20 bezeichnet, in der Datenbank 74 einander zugeordnet gespeichert werden. In diesem Falle ist der entsprechende Benutzer beim TAN-Server 70 akkreditiert. Stimmen die abgesandte Nachricht und die zurückgesandte Nachricht nicht überein, wird der entsprechende Benutzer nicht mit entsprechenden Einträgen in der Datenbank 74 registriert.

Für die Zwecke der Fernprogrammierung des Implantates 10 muss ein Arzt zusammen mit dem Erstellen eines Programmierauftrags oder zuvor eine TAN beim TAN-Server 70 abrufen. Dies kann entweder über die Fernprogrammieranwendung 58 dadurch geschehen, dass die TAN-Anfrage zunächst dem Servicecenter 30 übermittelt wird und dann von diesem über die Datenschnittstelle- 82 und die Datenkommunikationsschnittstelle 80 an den TAN-Server 70 übermittelt wird. Alternativ dazu kann die TAN-Anfrage auch direkt über die TAN-Serveranwendung 88 beim TAN-Server 70 erfolgen.

Nach Empfangen einer TAN-Anfrage erzeugt der TAN-Generator 76 des TAN-Servers 70 eine TAN und veranlasst, dass diese zum einen dem Patientengerät 20 übermittelt werden, welches die TAN dann im Speicher 24 speichert. Zum anderen wird die TAN über die Datenkommunikationsschnittstelle 86 an das Kommunikationsgerät 90 übersandt.

Ein Arzt, der mit Hilfe des Fernprogrammierclients 50 und der Fernprogrammieranwendung 58 einen Programmierauftrag für das Implantat 10 zusammenstellt, kann dem Programmierauftrag die seitens des Kommunikationsgerätes 90 empfangene TAN hinzufügen und über das Servicecenter 30 an das Patientengerät 20 senden.

Das Patientengerät 20 vergleicht die zusammen mit dem Programmierauftrag empfangene TAN mit der zuvor seitens des TAN-Servers 70 übermittelten und im Speicher 24 gespeicherten TAN. Im Falle der Übereinstimmung der beiden TAN wird der Programmierauftrag an das Implantat 10 weitergeleitet.

Die Anordnung umfasst somit die folgenden Komponenten:
- ein vom dem oder den das eigentliche Servicecenter 30 bildenden Servern physikalisch, administrativ und organisatorisch getrennter TAN-Server 70, benutzt durch eine eigene, separat lauffähige TAN-Serveranwendung 88.
- das Servicecenter 30, zugänglich über eine Fernprogrammieranwendung 58, die auch auf einem vom Servicecenter 30 entfernten Fernprogrammierclient 50, z. B. einem Arztrechner lauffähig ist.
- das Patientengerät 20 (mobiles Gerät, CardioMessenger) in der Nähe des Patienten.
- das fernprogrammierbare Implantat 10, implantiert im Patienten.

Eine Fernprogrammierung des Implantats 10 mittels dieser Anordnung läuft wie folgt ab. Zunächst akkreditiert sich ein Arzt für die Fernprogrammierung:
1. Zeitgleich mit dem Erhalt einer Benutzerkennung für das Servicecenter 30 erhält jeder Benutzer (Arzt) die Möglichkeit, sich separat auf einem speziell für die Fernprogrammierung (Umprogrammierung aus der Ferne) existierenden TAN-Server 70 zu akkreditieren und dort eine Nachrichtenadresse (Handy-Nummer, eine Email-Adresse oder eine Fax-Adresse o.ä.) zu hinterlegen.
2. Wählt der Arzt diese Möglichkeit und hinterlegt Handy-Nummer, e-mail-Adresse oder Fax-Nummer, so bekommt er eine Testnachricht dorthin gesendet, die er zur Verifizierung über eine TAN-Serveranwendung 88 zum TAN-Server 70 senden muss. Erst damit ist die hinterlegte Nachrichten-Adresse gültig.

Anschließend kann dieser Arzt das Implantat 10 umprogrammieren:
3. Sobald der Arzt jetzt die Notwendigkeit sieht, ein Implantat 10 aus der Ferne umzuprogrammieren, ruft er die Fernprogrammieranwendung 58 auf, wählt mit dieser das umzuprogrammierende Implantat 10 aus und signalisiert, dass er eine mobile TAN benötigt. Dieses Signal veranlasst er entweder in der Fernprogrammieranwendung 58 (die es über das Servicecenter 30 zu dem TAN-Server 70 weiterleitet) oder in der TAN-Serveranwendung 88 selbst.
4. Jetzt sendet der TAN-Server 70 eine zufällige TAN
   - an das Patientengerät 20
   - an die Geräteadresse, die der Arzt im TAN-Server 70 für sich hinterlegt hat.
5. Die Fernprogrammieranwendung 58 verlangt zur Bestätigung der Umprogrammierung des Implantats 10 aus der Ferne jetzt die TAN, die der Arzt auf seinem Kommunikationsgerät 90 erhalten hat.
6. Dem Programmierauftrag (Datenpaket), das die Umprogrammierungsanweisungen für das Implantat 10 enthält, wird diese TAN hinzugefügt.
7. Danach wird der Programmierauftrag durch das Servicecenter 30 an das Patientengerät 20 gesendet.
8. Bei Erhalt des Programmierauftrags überprüft das Patientengerät 20, ob die darin enthaltene TAN dieselbe ist wie die, die es vom TAN-Server 70 erhalten hat.
9. Ist das der Fall, sendet das Patientengerät 20 den Programmierauftrag weiter zum Implantat 10. Anderenfalls verwirft es den Programmierauftrag.

## Patentansprüche

1. Anordnung für die Fernprogrammierung eines programmierbaren persönlichen medizinischen Gerätes (10, 20), insbesondere eines implantierbaren medizinischen Gerätes (10), wie ein Herzschrittmacher, Defibrillator oder dergleichen, mit den Komponenten:
- TAN-Server (70),
- persönliches Gerät (10, 20)
von denen der TAN-Server (70) aufweist:
eine Datenbank (74), die einander zugeordnete Einträge für eine Benutzerkennung, ein der Benutzerkennung zugeordnetes persönliches Gerät, **gekennzeichnet durch** eine Gerätekennung und eine der Benutzerkennung zugeordnete Nachrichten-Adresse eines adressierbaren Kommunikationsgerätes (90) enthält,
eine TAN-Benutzerschnittstelle (88, 60, 62, 84), die ausgebildet ist, Eingaben eines Benutzers entgegenzunehmen und die Eingaben einer Benutzerkennung zuzuordnen und derart zugeordnet an den TAN-Server (70) weiterzuleiten,
eine Nachrichten-Schnittstelle (86), über die der TAN-Server (70) eine Nachricht an ein adressierbares Kommunikationsgerät (90) (z. B. Fax, e-mail, Handy (SMS)) eines Benutzers, dass **durch** eine Nachrichten-Adresse identifiziert ist, senden kann und
eine Datenkommunikationsschnittstelle (80) für eine wenigstens eine mittelbare Verbindung zur Übertragung von Daten an das programmierbare persönliche Gerät (10, 20)
und ausgebildet ist:
zum Zwecke der TAN Anfrage
eine einer Benutzerkennung zugeordnete TAN Anfrage zu empfangen und
anschließend eine TAN-Nachricht zu generieren und
- über die Nachrichten-Schnittstelle (86) an das **durch** die über die Datenbank-Einträge der Benutzer-Kennung des anfragenden Benutzers zugeordnete adressierbare Kommunikationsgerät (90) und
- über die Datenkommunikationsschnittstelle (80) an ein **durch** die Datenbank-Einträge der Benutzer-Kennung zugeordnetes, **durch** seine Geräte-Kennung identifiziertes persönliches Gerät (10, 20) eine TAN
zu versenden, und
von denen das programmierbare persönliche Gerät (10, 20)
eine Datenkommunikationsschnittstelle (28) zum wenigstens mittelbaren Verbinden des persönlichen programmierbaren Gerätes (10, 20) mit dem TAN-Server (70) aufweist,
einen TAN-Speicher (14, 24) für eine seitens des TAN-Servers (70) empfangene TAN besitzt und ausgebildet ist, eine seitens des TAN-Servers (70) empfangene TAN in dem TAN-Speicher (14, 24) zu speichern und
eine programmierbare Steuerung (12, 22) für Funktionen des persönlichen Gerätes (10, 20) aufweist.

2. Anordnung nach Anspruch 1, **dadurch gekennzeichnet, dass**
der TAN-Server (70) weiterhin ausgebildet ist, zum Zwecke der Akkreditierung eines Benutzers:
- über die TAN-Benutzerschnittstelle (88, 60, 62, 84) eine Benutzer-Kennung und eine Nachrichten-Adresse eines Kommunikationsgerätes (90) zu empfangen und daraufhin eine Nachricht an das Kommunikationsgerät (90) zu versenden,
- über die TAN-Benutzerschnittstelle (88, 60, 62, 84) eine Nachricht zu empfangen, diese Nachricht mit der an das Kommunikationsgerät (90) gesandten Nachricht zu vergleichen und
- im Falle der Übereinstimmung die Benutzer-Kennung zusammen mit der Nachrichten-Adresse des Kommunikationsgerätes (90) als einander zugeordnete Einträge in der Datenbank (74) zu speichern.

3. Anordnung nach Anspruch 1 oder 2, **dadurch gekennzeichnet, dass** die TAN-Benutzerschnittstelle (88, 60, 62, 84) des TAN-Servers (70) mit einer TAN-Serveranwendung (88) verbunden ist, die dazu ausgebildet ist, eine TAN-Anfrage eines Benutzers unter einer Zuordnung zur Benutzerkennung entgegenzunehmen und an den TAN-Server (70) weiterzugeben.

4. Anordnung nach einem der Ansprüche 1 bis 3, **dadurch gekennzeichnet, dass** die TAN-Serveranwendung (88) auf einem von dem TAN-Server (70) räumlich entfernten Computer lauffähig ist und über ein Datennetz mit dem TAN-Server (70) verbunden ist.

5. Anordnung nach einem der Ansprüche 1 bis 4, **dadurch gekennzeichnet, dass** die Anordnung als weitere Komponente ein Servicecenter (30) aufweist, das
eine erste Datenkommunikationsschnittstelle (36) für eine Datenkommunikation mit dem persönlichen Gerät (10, 20) aufweist sowie
eine weitere, mit dem Servicecenter (30) verbundene Service-Benutzerschnittstelle (58, 60, 62, 38) besitzt, die es erlaubt, Programmieraufträge für das programmierbare persönliche Gerät (10, 20) zusammenzustellen, ein Absenden eines zusammengestellten Programmierauftrags auszulösen und die ausgebildet ist eine manuelle Eingabe einer TAN zu einem Programmierauftrag abzufragen und eine entgegengenommene TAN einem Programmierauftrag hinzuzufügen,
und das ausgebildet ist, einen abgesendeten Programmierauftrag zusammen mit einer TAN an das persönliche Gerät (10, 20) zu senden,
wobei das persönliche Gerät (10, 20) dazu ausgebildet ist, eine seitens des TAN-Servers (70) empfangene, gespeicherte TAN mit einer zusammen mit einem Programmierauftrag seitens des Servicecenters (30) empfangenen TAN zu vergleichen und den Programmierauftrag nur auszuführen oder an ein Implantat (10) weiterzuleiten, wenn beide TAN identisch sind.

6. Anordnung nach Anspruch 5, **dadurch gekennzeichnet, dass** die Service-Benutzerschnittstelle (58, 60, 62, 38) als auf einem vom Servicecenter (30) entfernt lauffähige Fernprogrammieranwendung (58) ausgebildet oder mit einer solchen verbunden ist.

7. Anordnung nach Anspruch 5 oder 6, **dadurch gekennzeichnet, dass** die Service-Benutzerschnittstelle (58, 60, 62, 38) ausgebildet ist, eine TAN-Anfrage eines Benutzers zusammen mit einer Benutzerkennung dieses Benutzers entgegenzunehmen, wobei das Servicecenter (30) eine weitere Datenschnittstelle (82) aufweist, die ausgebildet ist einen Datenaustausch vom Servicecenter (30) zum TAN-Server (70) zu ermöglichen und das Servicecenter (30) ausgebildet ist, eine über die Service-Benutzerschnittstelle (58, 60, 62, 38) empfangene TAN-Anfrage an den TAN-Server (70) weiterzuleiten.

8. TAN-Server (70) für eine Anordnung gemäß der Ansprüche 1 bis 7, **dadurch gekennzeichnet, dass** der TAN-Server (70)
eine Datenbank (74) aufweist, die einander zugeordnete Einträge für eine Benutzerkennung, ein der Benutzerkennung zugeordnetes persönliches Gerät (10, 20), **gekennzeichnet durch** eine Gerätekennung und eine der Benutzerkennung zugeordnete Nachrichten-Adresse eines adressierbaren Kommunikationsgerätes (90) enthält, sowie
eine TAN-Benutzerschnittstelle (88, 60, 62, 84), die ausgebildet ist Eingaben eines Benutzers entgegenzunehmen und die Eingaben einer Benutzerkennung zuzuordnen und derart zugeordnet an den TAN-Server (70) weiterzuleiten,
eine Nachrichten-Schnittstelle (86), über die der TAN-Server (70) eine Nachricht an ein adressierbares Kommunikationsgerät (90) (z. B. Fax, e-mail, Handy (SMS)) eines Benutzers, dass **durch** eine Nachrichten-Adresse identifiziert ist, senden kann und
eine Datenkommunikationsschnittstelle (80) für eine wenigstens eine mittelbare Verbindung zur Übertragung von Daten an das programmierbare persönliche Gerät (10, 20)
und ausgebildet ist:
zum Zwecke der TAN Anfrage
eine einer Benutzerkennung zugeordnete TAN-Anfrage zu empfangen und
anschließend eine TAN-Nachricht zu generieren und
- über die Nachrichten-Schnittstelle (86) an das **durch** die über die Datenbank-Einträge der Benutzerkennung des anfragenden Benutzers zugeordnete adressierbare Kommunikationsgerät (90) und
- über die Datenkommunikationsschnittstelle (80) an ein **durch** die Datenbank-Einträge der Benutzerkennung zugeordnetes, **durch** seine Gerätekennung identifiziertes persönliche Geräte (10, 20) eine TAN zu versenden.

9. TAN-Server (70) nach Anspruch 8, **dadurch gekennzeichnet, dass**
der TAN-Server (70) weiterhin ausgebildet ist, zum Zwecke der Akkreditierung eines Benutzers:
- über die TAN-Benutzerschnittstelle (88, 60, 62, 84) eine Benutzerkennung und eine Nachrichten-Adresse eines Kommunikationsgerätes (90) zu empfangen und daraufhin eine Nachricht an das Kommunikationsgerät (90) zu versenden,
- über die TAN-Benutzerschnittstelle (88, 60, 62, 84) eine Nachricht zu empfangen, diese Nachricht mit der an das Kommunikationsgerät (90) gesandten Nachricht zu vergleichen und
- im Falle der Übereinstimmung die Benutzerkennung zusammen mit der Nachrichten-Adresse des Kommunikationsgerätes (90) als einander zugeordnete Einträge in der Datenbank (74) zu speichern.

10. TAN-Server (90) nach Anspruch 8 oder 9, **dadurch gekennzeichnet, dass** die TAN-Benutzerschnittstelle (88, 60, 62, 84) des TAN-Servers (70) mit einer TAN-Serveranwendung (88) verbunden ist, die dazu ausgebildet ist, eine TAN-Anfrage eines Benutzers unter einer Zuordnung zur Benutzerkennung entgegenzunehmen und an den TAN-Server (70) weiterzugeben.

11. Servicecenter (30) für eine Anordnung gemäß der Ansprüche 1 bis 7, **dadurch gekennzeichnet, dass** das Servicecenter (30) eine erste Datenkommunikationsschnittstelle (36) für eine Datenkommunikation mit dem persönlichen Gerät (10, 20) aufweist sowie
eine mit dem Servicecenter (30) verbundene Service-Benutzerschnittstelle (58, 60, 62, 38) besitzt, die es erlaubt, Programmieraufträge für ein dem Servicecenter (30) zugeordnetes programmierbares persönliches Gerät (10, 20) zusammenzustellen, ein Absenden eines zusammengestellten Programmierauftrags auszulösen und die ausgebildet ist eine manuelle Eingabe einer TAN zu einem Programmierauftrag abzufragen und eine entgegengenommene TAN einem Programmierauftrag hinzuzufügen,
und das ausgebildet ist, einen abgesendeten Programmierauftrag zusammen mit einer TAN an das persönliche Gerät (10, 20) zu senden

12. Servicecenter (30) nach Anspruch 11, **dadurch gekennzeichnet, dass** die Benutzerschnittstelle als Fernprogrammieranwendung (58) ausgebildet, die in Abhängigkeit einer Fernprogrammierservereinheit (40) auf einem von dem Servicecenter (30) entfernten Fernprogrammier-Client (50) lauffähig ist und die ausgebildet ist, eine Programmierung mehrerer unterschiedlicher persönlicher Geräte (10, 20) zu erlauben und eine Service-Benutzerschnittstelle (58, 60, 62, 38) zur Auswahl eines persönlichen Gerätes (10, 20) und zum Zusammenstellen eines Programmierauftrags für ein jeweils ausgewähltes persönliches Gerät (10, 20) sowie zur Anfrage einer dem persönlichen Gerät (10, 20) zugeordneten TAN aufweist,
wobei die Fernprogrammieranwendung (58) weiter ausgebildet ist, einen Programmierauftrag zusammen mit einer Gerätekennung und einer TAN über das Servicecenter (30) an ein persönliches Gerät (10, 20) zu senden.

13. Persönliches Gerät (10, 20) für eine Anordnung gemäß der Ansprüche 1 bis 7, **dadurch gekennzeichnet, dass** das persönliche Gerät ein Patientengerät (20) ist, das einem Implantat (10) eines Patienten fest zugeordnet ist und das
eine Datenkommunikationsschnittstelle (28) zum wenigstens mittelbaren Verbinden des persönlichen programmierbaren Gerätes (10, 20) mit dem TAN-Server (70) aufweist, sowie
einen TAN-Speicher (14, 24) für eine seitens des TAN-Servers (70) empfangene TAN besitzt und ausgebildet ist, eine seitens des TAN-Servers (70) empfangene TAN in dem TAN-Speicher (14, 24) zu speichern und
eine programmierbare Steuerung (12, 22) für Funktionen des Patientengerätes (20),
und das ausgebildet ist eine seitens des TAN-Servers (70) empfangene, gespeicherte TAN mit einer zusammen mit einem Programmierauftrag seitens eines Servicecenters (30) empfangenen TAN zu vergleichen und den Programmierauftrag nur auszuführen oder an ein Implantat (10) weiterzuleiten, wenn beide TAN identisch sind.

14. Verfahren zum Fernprogrammieren eines Patientengerätes (20) oder eines einem Patientengerät (20) zugeordneten aktiven medizinischen Implantats (10), mit den folgenden Verfahrensschritten für das Erzeugen und Verteilen einer TAN:
- Empfangen einer TAN-Anfrage enthaltend eine Benutzerkennung und eine Geräte-Kennung
- Generieren einer TAN
- Versenden der generierten TAN an ein durch die Geräte-Kennung identifiziertes
Patientengerät (20) oder Implantat (10) und an einer der Benutzerkennung zugeordnete Nachrichten-Adresse eines Kommunikationsgerätes (90) des Benutzers.

15. Verfahren nach Anspruch 14, mit den folgenden Verfahrensschritten für das Akkreditieren eines Benutzers:
- Empfangen einer Benutzerkennung und einer Nachrichtenadresse
- Versenden einer Nachricht an die Nachrichtenadresse
- Empfangen einer Nachricht, die eine Benutzkennung enthält,
- Vergleichen der versandten Nachricht mit der mit der empfangenen Nachricht, und
- im Falle der Übereinstimung: Speichern der Benutzerkennung samt zugeordneter Nachrichtenadresse,
- andernfalls: Zurückweisen der Benutzerkennung.
